# EUROPEAN PATENT APPLICATION

(11) **EP 1 618 826 A1**
(43) Date of publication of application: **25.01.2006**
(21) Application number: 04726027.8
(22) Date of filing: 06.04.2004
(51) Int. Cl.: A61B 1/00

(54) **MOUTHPIECE AND ORAL CAVITY PHOTOGRAPHING SYSTEM USING THAT MOUTHPIECE**

(30) Priority: 07.04.2003 JP 2003103075
(71) Applicant: Olympus Corporation, Tokyo 151-0072 (JP)
(72) Inventor: KOBAYASHI, Hiroyoshi, -cho Hachioji-shi Tokyo 192-8512 (JP)
(74) Representative: von Hellfeld, Axel
(86) International application number: PCT/JP2004/004965
(87) International publication number: WO 2004/089194

(57) **Abstract**

A mouthpiece is used for a mouth photographing apparatus for photographing the oral cavity of a patient by shading external light. A fitting portion is used for fitting a shading unit of the mouth photographing apparatus when taking a picture. A biting portion is formed to position the mouthpiece close to the teeth of a patient by being bitten by the patient with the teeth of upper and lower jaws.

## Description

### Technical Field

The present invention relates to a mouth photographing apparatus for observing a mouth and a mouthpiece used for the mouth photographing apparatus.

### Background Art

In recent years, a video scope using a CCD has been used for observing a mouth. On the other hand, it has been attempted to recognize correct colors by using a sensor such as a photodiode to measure the reflection intensity of illumination light.

For example the Jpn. Pat. KOKAI. Publication No. 11-47092 discloses an application of using an LED as a light source for such observation. A white LED is lit to measure the inside of the mouth of a patient after inserting a CCD into the mouth.

The Jpn. Pat. KOKAI. Publication No. 2001-212081 discloses a method of photographing the oral cavity of a patient, in which a camera unit is provided in a part of a mouth opener.

The photographing apparatus disclosed in the above Jpn. Pat. KOKAI. Publication No. 11-47092 emits a white LED light directly to an object surface. The amount of light is optically determined by the emitting distance and the light-emitting characteristic of the LED. However, though the camera unit is directly inserted into the mouth of a patient, a mouthpiece (a mouth opener, etc.) for ensuring observation is not disclosed.

The photographing apparatus disclosed in the Jpn. Pat. KOKAI. Publication No. 2001-212081 has a camera unit in a part of a mouth opener to open widely the lips of a patient by the mouth opener when taking a picture. However, the influence of external light is unavoidable in open space, light with different wavelengths is emitted to an object surface, and the reflection intensity of the light is not exactly measured.

### Disclosure of Invention

It is an object of the present invention to provide a mouthpiece and a photographing apparatus, which can observe exactly the oral cavity of a patent and minimize the load of a patient.

According to a first aspect of the present invention, there is provided a mouthpiece used for a mouth photographing apparatus for photographing an oral cavity of a patient by shading external light, comprising:
a fitting portion which is used for fitting a shading means of the mouth photographing apparatus when taking a picture; and
a biting portion which positions the mouthpiece close to the teeth of a patient by being bitten by the patient with the teeth of upper and lower jaws.

According to a second aspect of the present invention, there is provided a mouthpiece according to the first aspect, wherein the fitting portion has the characteristic to transmit substantially no light.

According to a third aspect of the present invention, there is provided a mouthpiece according to the first aspect, further comprising a shading cover which adjoins to the fitting portion and presses the lips of a patient, and serves as a guide when the shading means of the mouth photographing apparatus is applied to the fitting portion, wherein the shading cover has the characteristic to transmit substantially no external light.

According to a fourth aspect of the present invention, there is provided a mouthpiece according to the first aspect, wherein the biting portion is provided in two pieces on the left and right sides to be bitten by the upper/lower and left/right back teeth of a patient, and the circumference from the left to right biting portions is approximately 50 - 90 mm.

According to a fifth aspect of the present invention, there is provided a mouthpiece according to the first aspect, wherein the fitting portion is made of styrene group elastomer resin with the rubber hardness of approximately 20 - 70.

According to a sixth aspect of the present invention, there is provided a mouthpiece according to the third aspect, wherein the shading cover is made of styrene group elastomer resin with the rubber hardness of approximately 10 - 40.

According to a seventh aspect of the present invention, there is provided a mouthpiece according to the second aspect, wherein the color of at least the fitting portion is black.

According to an eighth aspect of the present invention, there is provided a mouth photographing apparatus for photographing the oral cavity of a patient by using the mouthpiece according to any one of the first to seventh aspects, comprising:
a lighting means to light the teeth of a patient;
a photographing means to photograph the teeth of a patient lit by the lighting means; and
a shading means to shade the photographing means from external light;
wherein the lighting means is positioned at a location capable of lighting the teeth of a patient substantially evenly by fitting the shading means to the fitting portion of the mouthpiece; and
the photographing means is positioned at a location capable of photographing the teeth of a patient in the state substantially focused.

According to a ninth aspect of the present invention, there is provided a mouth photographing apparatus according to the eighth aspect, wherein the lighting means and photographing means are configured as one body and rotatably about a predetermined rotation axis.

According to a tenth aspect of the present invention, there is provided a mouth photographing apparatus according to the ninth aspect, further comprising:
a turning mechanism means to turn the lighting means and photographing means configured as one body; and
an operation means to adjust the turning amount of the turning mechanism means.

According to an eleventh aspect of the present invention, there is provided a mouth photographing apparatus according to the tenth aspect, wherein the operation means is composed of a jog wheel and a wire moved by the rotation of the jog wheel, and the turning mechanism means turns the lighting means and photographing means configured as one body according to the movement of the wire.

### Brief Description of Drawings

FIG. 1 is a conceptual illustration showing the configuration when a camera unit 1 emits an LED light to a part of the teeth of a patient 500 to taking a picture;
FIGS. 2A - 2D are conceptual illustrations showing the fitting of a mouthpiece 20;
FIG. 3 is a block diagram of the whole system configuration of a mouth photographing apparatus including the camera unit 1;
FIG. 4A is a sectional view of the camera unit 1;
FIG. 4B is a schematic diagram of an LED board 7;
FIG. 5 is a partially detailed view of the optical system of the camera unit 1;
FIG. 6 is a graph showing the wavelength bands (8 bands) 101 of the wavelengths of the output light of LEDS 7a - 7h, and the wavelength band 100 of a CCD filter;
FIGS. 7A - 7D are views for explaining a second embodiment of the present invention;
FIG. 8A is a table showing the experiment results on the rubber hardness of a mouthpiece 20;
FIG. 8B is a table showing the experiment results on the rubber hardness of a shading cover 20c;
FIGS. 9A - 9D are views for explaining a third embodiment of the present invention;
FIG. 10 is a view showing the configuration of a camera unit 1 according to a fourth embodiment of the present invention;
FIGS. 11A and 11B are views for explaining the effects of the fourth embodiment of the present invention; and
FIGS. 12A and 12B are views showing the configuration of a camera unit 1 according to a fifth embodiment of the present invention.

### Best Mode for Carrying Out the Invention

Embodiments of the present invention will be explained in detail hereinafter with reference to the accompanying drawings.

### (Embodiment 1)

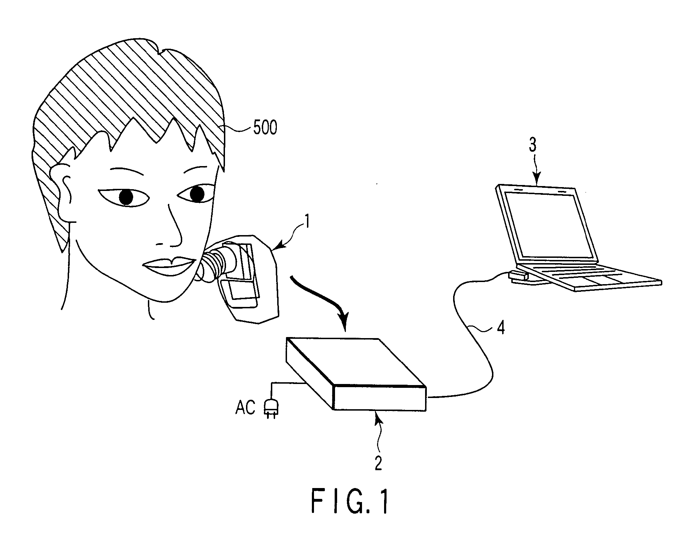

FIG. 1 is a conceptual illustration showing the configuration when a mouth photographing apparatus (hereinafter called a camera unit) 1 emits an LED light to a part of the teeth of a patient 5 to when taking a picture. The camera unit 1 is assumed to measure correctly the color of the teeth of the patient 500.

The image data on the color of the teeth of the patient 500 obtained by the camera unit 1 is applied to a personal computer (hereinafter called a PC) 3 through a cradle 2 and USB (universal serial bus) 2 interface 4, and analyzed there. An observer analyzes the color data of the photographed image, and measures the color of the teeth of the patient 500 for spatial color coordinates.

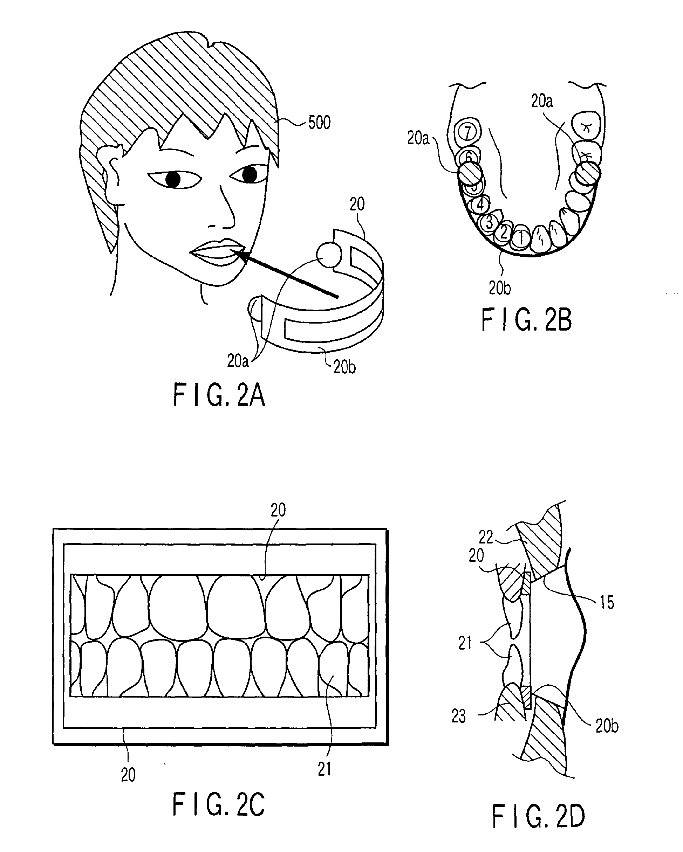

FIGS. 2A - 2D are conceptual illustrations showing the fitting of a mouthpiece 20. The mouthpiece 20 is used for the mouth photographing apparatus for photographing the oral cavity of a patient by shading external light. The mouthpiece has a mouthpiece fitting portion 20b having the characteristic of transmitting substantially no light to fit a shading means (a shading frame 15 described later) of the camera unit, and a biting portion 20a of a mouthpiece is bitten by the teeth 20 of the upper jaw and teeth 21 of the lower jaw of a patient 500 to position the mouthpiece 20 close to the teeth 21 of the patient 500.

FIG. 2A is a view showing that the patient 500 is going to fit the mouthpiece 20. FIG. 2B is a view showing the position of the biting portion 20a of the mouthpiece when the mouthpiece 20 is fit. FIG. 2C is a view from the front side showing the teeth 21 of the patient 500 fit with the mouthpiece 20. FIG. 2D is a sectional view of the teeth 21 of the patient 500 fit with the mouthpiece 20. In FIG. 2D, a reference numeral 22 denotes the lips of the patient 500, and 23 denotes the gums of the patient 500.

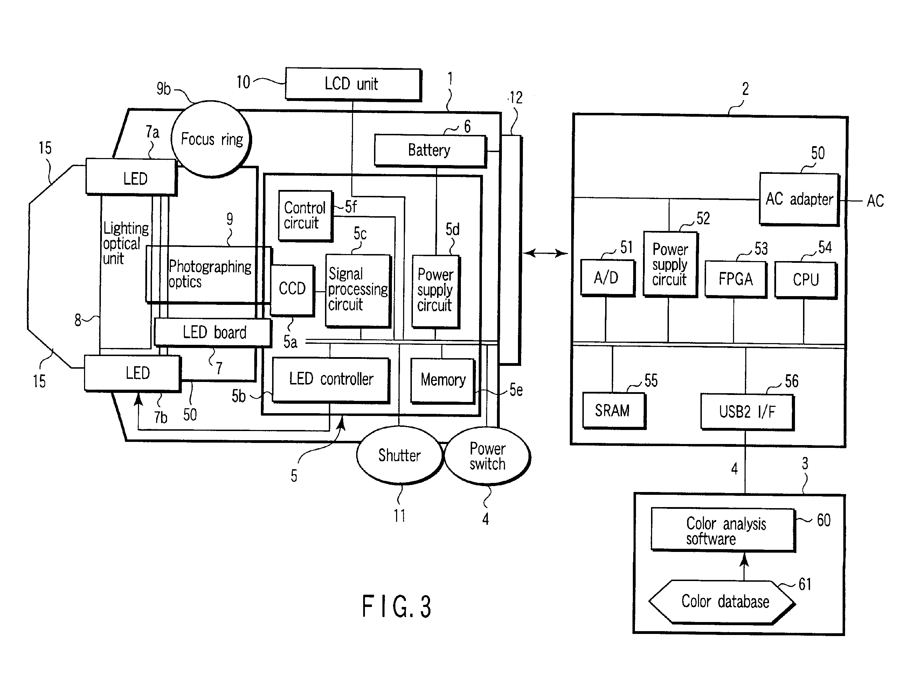

FIG. 3 is a block diagram of the whole system configuration of a mouth photographing apparatus including the camera unit 1. In FIG. 3, an electric board 5 of the camera unit 1 has a CCD 5a having a color filter, an LED controller 5b, a signal processing circuit 5c, a power supply circuit 5d, a memory 5e, and a control circuit 5f. A mirror frame 50 includes a photographing optics 9, and a lighting optical unit 8. LEDS 7a - 7h (only 7a and 7b are shown in FIG. 3) are arranged on the circumference of a disc-shaped LED board 7. The camera unit 1 has a shading frame (shading means) 15 at the front end, and a focus ring 9b, LCD unit 10, contact 12, power switch 4 and shutter 11 on the outside surface.

A cradle 2 includes an AC adapter 50, an A/D converter 51, a power supply circuit 52, a FPGA 53, a CPU 54, a SRAM 55, an USB 2, and an I/F 56. The LEDS 7a - 7h and lighting optical unit 6 constitute a lighting means, and the photographing optics 9 and CCD 5a constitute a photographing means.

A color analysis software 60 is installed in the PC 3, and a color database 61 is built up.

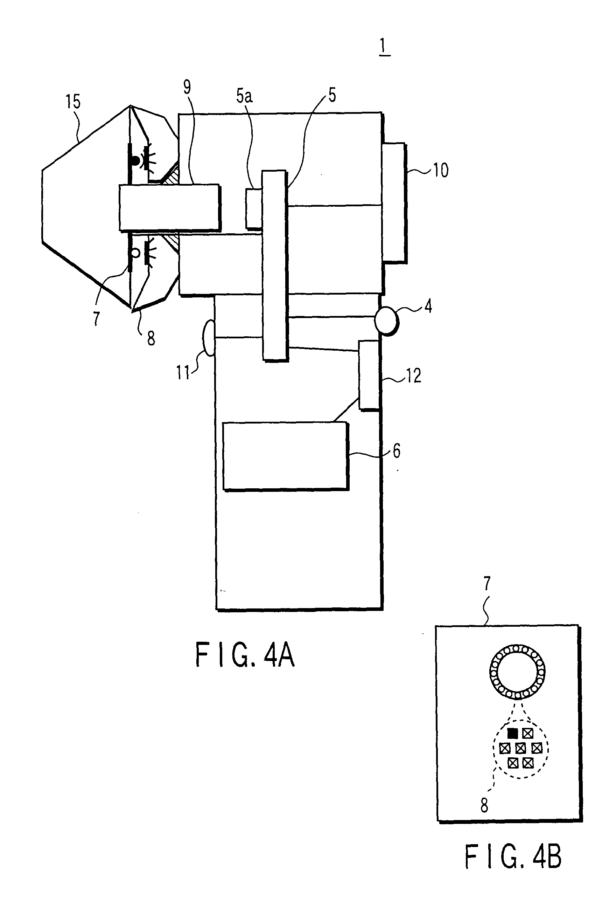

FIG. 4A is a sectional view of the camera unit 1. FIG. 4B is a schematic diagram of the LED board 7.

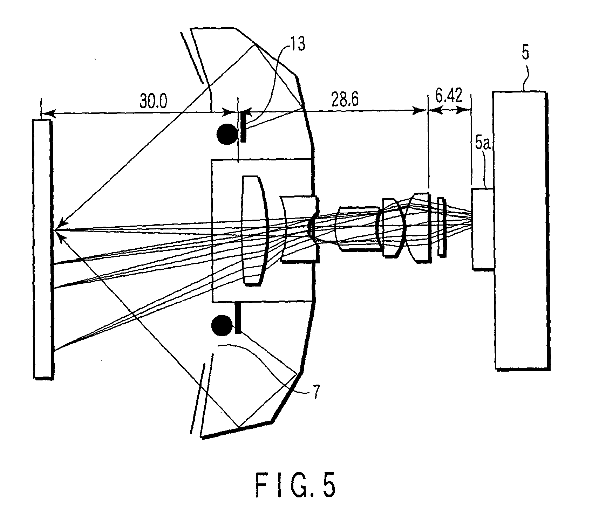

FIG. 5 is a partially detailed view of the optical system of the camera unit 1, showing various optical elements including an optical sheet 13 having the photodiffusion effect.

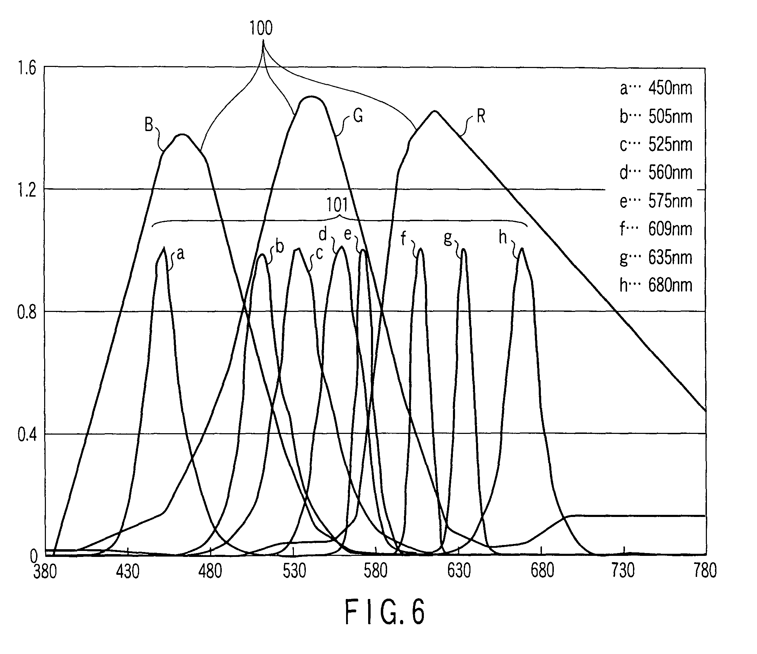

FIG. 6 is a graph showing the wavelength bands (8 bands) 101 of the wavelengths of the output light of LEDS 7a - 7h, and the wavelength band 100 of a CCD filter.

In the above configuration, as shown in FIG. 2, the patient 500 bites the mouthpiece 20, so that the biting portion 20a of the mouthpiece is positioned at the boundary of the teeth 5 and 6. After the biting position is decided, the observer lets the patient 500 hold the shading frame 15 with the lips 22 opposite to a desired tooth (not shown), and turns on the power switch 4 to power the camera unit 1. When the power switch is turned on, the circuit components on the electric board 5, that is, the CCD 5a, LED controller 5b, signal processing circuit 5c, power supply circuit 5d, memory 5e, control circuit 5f, and LCD unit 10 are driven by the power supplied from a battery 6.

At the same time, a current is supplied to the LED board 7 by the instruction from the control unit 5f and LED controller 5b, and the LEDS 7a - 7h are lit. As only a preset LED is lit, the amount of light can be controlled by driving with small current for saving power. It is also possible to provide two or more LEDS in one LED package. As a result, the light from LEDS 7a - 7h is completely diffused in the lighting optical unit 8 (a light-diffusing element) through the optical sheet 13, and applied evenly to the teeth 21 of the patient 500.

The observer applies the camera unit 1 to the mouthpiece fitting portion 20b of the mouthpiece 20 at the position of the object tooth, adjusts the focus by the focus ring 9b provided in the photographing optics 9 to permit photographing from a near distance while monitoring the LCD unit 10, determines the subject position, and presses the shutter 11. The control circuit 5f responds to the depression of the shutter 11, and instructs a measurement mode.

According to the instruction from the LED controller 5b, a current is supplied to the LED board 7, and the light of eight wavelengths from LED 7a - 7h are repeatedly turned on and off at intervals of 1/30 seconds. The light applied to the teeth 21 of the patient 500 is reflected from the teeth 21, and taken into the photographing optics 9 to form an image in the CCD 5a. The electric signal converted photoelectrically by the CCD 5a is taken into the signal processing circuit 5c as an image data corresponding to the turning on/off of the light of eight wavelengths from LED 7a - 7h, and stored in the memory 5e.

After the measurement is completed, the observer connects the contact 12 of the camera unit 1 to the contact of the cradle 2 (not shown). The image data taken in the camera unit 1 is sent to the cradle 2.
The cradle 2 processes the received signal, and sends the image data to the PC 3 through the USB 2 (4). The PC 3 analyzes the color of the image data by using the color analysis software 60 and color database 61, and recognizes the color of the teeth 21 of the patient 500.

The camera unit 1 is supplied with electricity from the power supply system of the cradle 2 through the contact of the cradle 2 while the connection with the cradle 2 is held, and the electricity is stored in the internal battery 6.

### (Embodiment 2)

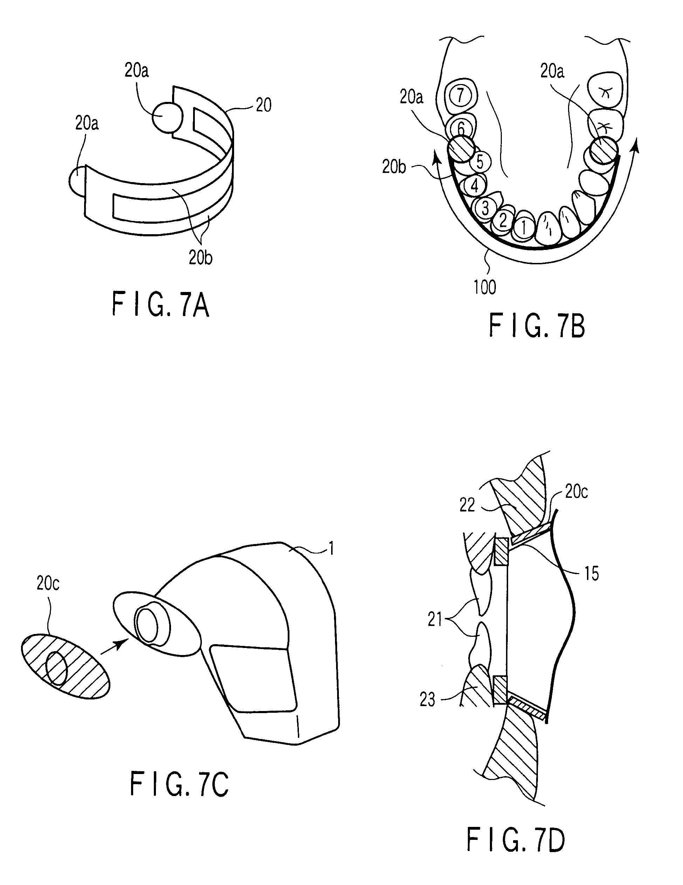

FIGS. 7A - 7D are views for explaining a second embodiment of the present invention. FIG. 7A is an external view of a mouthpiece 20. FIG. 7B shows the position of the biting portion 20a of the mouthpiece when the mouthpiece 20 is fit. FIG. 7C shows the camera unit 1 going to be fit with a shading cover 20c having the characteristic of transmitting substantially no external light. FIG. 7D is a sectional view when the mouthpiece 20 is fit to the teeth 21 of the patient 500. The mouthpiece 20 is made of black rubber made of elastomer group resin with the hardness of 20 - 70. In an elastomer group resin, the rubber hardness can be adjusted by additives.

It is desirable to fit the mouthpiece 20 along the alignment of the teeth 21 of the patient 500 when measuring the inside of mouth. Therefore, the mouthpiece is preferably made of material with the low rubber hardness (soft). Further, the mouthpiece 20 is desirably not deformed to apply the camera unit 1 stably to the patient. Therefore, the mouthpiece is preferrably made of material with the high rubber hardness (hard).

When the patient 500 bites the biting portion 20a of the mouthpiece to fix the mouthpiece 20, if the rubber hardness of the mouthpiece is low (soft), the mouthpiece 20 is largely deformed when being bitten, and becomes unstable. Contrarily, if the rubber hardness is high (hard), the mouthpiece is hard to bite.

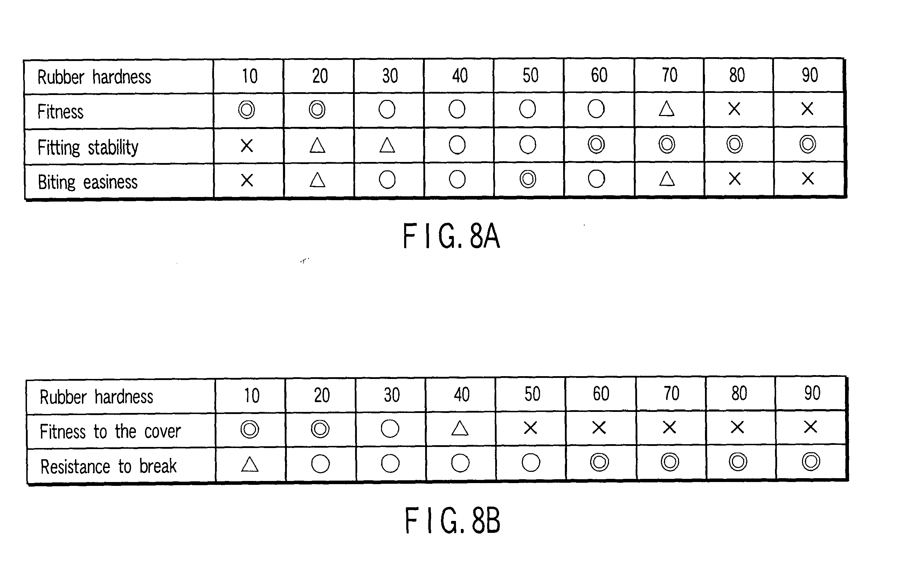

FIG. 8A is a table showing the experiment results on the rubber hardness of the mouthpiece 20. Symbols used in the table mean: ⓞ...Optimum, ○...Suitable, Δ...Barely suitable, ×...Unsuitable. As seen from the table, the rubber hardness of the mouthpiece 20 is suitable in a range of 20 - 70, desirably 40 - 60.

The distance from the left to right biting portions 20a of the mouthpiece (the circumference 100 of the mouthpiece) is 50 - 90 mm in the mouthpiece 20 in order to cover the upper and lower gums. The patient 500 can stably bite the relatively flat teeth 4, 5, 6. Especially, the teeth 5 and 6 can be bitten most easily. The back teeth 6 and 7 can be stably bitten. But, if a foreign matter is inserted close to the throat, the patient suffers from nerves and feels sick. Thus, the tooth 7 is undesirable for holding the mouthpiece 20. The results of measuring 200 samples of the circumference from the left-side teeth 5, 6 to the right-side teeth 5, 6 of 16 to 60 years old people are 55 - 95 mm in a range of satisfying the statistics ±3σ (99%).

Examining the above facts, the mouthpiece 20 can be fit to most patient 500 by setting the circumference to 50 - 90 mm (with an error of ±5 mm). Actually, the biting of teeth 4, 5 and 6 can be fixed by preparing the mouthpiece 20 with the circumference 100 varied in three steps of 60, 75 and 90. Of course, the mouthpiece 20 becomes to be easier to bite by setting the circumference in more steps.

When the patient 500 fits the mouthpiece 20, fix the mouthpiece by biting the biting portion 20a by the relatively flat teeth 4, 5 and 6. The fitting portion 20b of the mouthpiece 20 covers the gums of the patient 500 to enable observation of only the tooth 21. Further, the mouthpiece 20 is made of relatively soft flexible material with the rubber hardness of 20 - 70, and the mouthpiece is easy to fit the alignment of the tooth 21, and the biting portion 20a is easy to bite. The mouthpiece 20 is black, and directly reflects an illumination light and does not cause a stray light in the photographing optics 9.

Further, the shading cover 20c made of elastomer group resin with the rubber hardness of 10 - 40 can be attached to the outside of the shading frame 15 provided at the front end of the camera unit 1. Low rubber hardness (soft) is suitable to fit the shading cover 20c to the shading frame 15 of the camera unit 1. Contrarily, high rubber hardness (hard) is suitable to make the shading cover hard to break to the shading frame 15 of the camera unit 1.

FIG. 8B is a table showing the experiment results on the rubber hardness of the shading cover 20c. Symbols used in the table mean: ⊚...Optimum, ○...Suitable, Δ...Barely suitable, ×...Unsuitable. As seen from the table, the rubber hardness of the shading cover 20c is suitable in a range of 10 - 40, desirably 20 - 30.

The observer applies the shading cover 20c to an observing tooth in the fitting portion 20b of the mouthpiece 20, to be opposite to the lighting optical unit 8, photographing optics 9 and CCD 5a of the camera unit 1. In this time, the patient 500 adjusts the fitness through the mouthpiece 20. The patient 500 simply holds the mouthpiece with the lips and needs not open the mouth widely.

### (Embodiment 3)

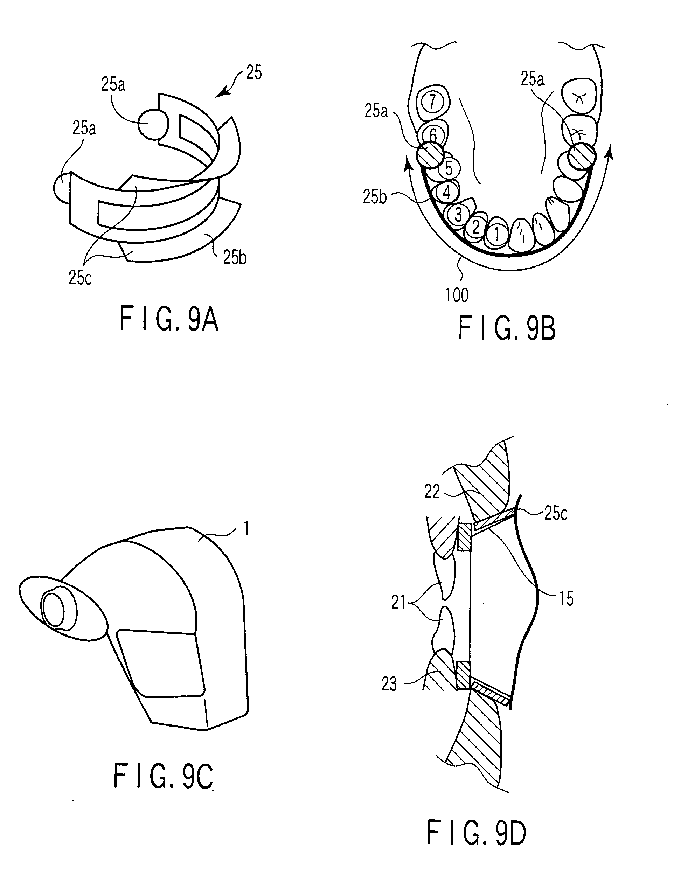

FIGS. 9A - 9D are views for explaining a third embodiment of the present invention. FIG. 9A is an external view of a mouthpiece 25. FIG. 9B shows the position of the biting portion 25a of the mouthpiece when the mouthpiece 25 is fit. FIG. 9C is an external view of the camera unit 1. FIG. 9D is a sectional view when the mouthpiece 25 is fit to the teeth 21 of the patient.

The mouthpiece 25 is configured as one unit with the shading cover 25c (FIG. 9D), so that the shading frame 15 provided at the front end of the camera unit 1 does not directly touch the lips 22 of the patient.
The mouthpiece 25 is made of relatively soft material with the hardness of 20 - 70, and fits easily to the shading frame 15 of the camera unit 1.

The mouthpiece 25 is made of styrene group elastomer resin. The observer applies the mouthpiece 25 to an observing tooth in the fitting portion 25b of the mouthpiece 25, to be opposite to the lighting optical unit 8, photographing optics 9 and CCD 5a of the camera unit 1, and take a picture. When the mouthpiece 25 is fit to the shading frame 15, the mouthpiece 25 has the functions of protecting the gums of the patient and preventing patient's lips from being directly touch by the shading frame 15. Therefore, the photographing cost is lower than the configuration with two parts, the mouthpiece 20 and shading cover 25c, as shown in the second embodiment.

### (Embodiment 4)

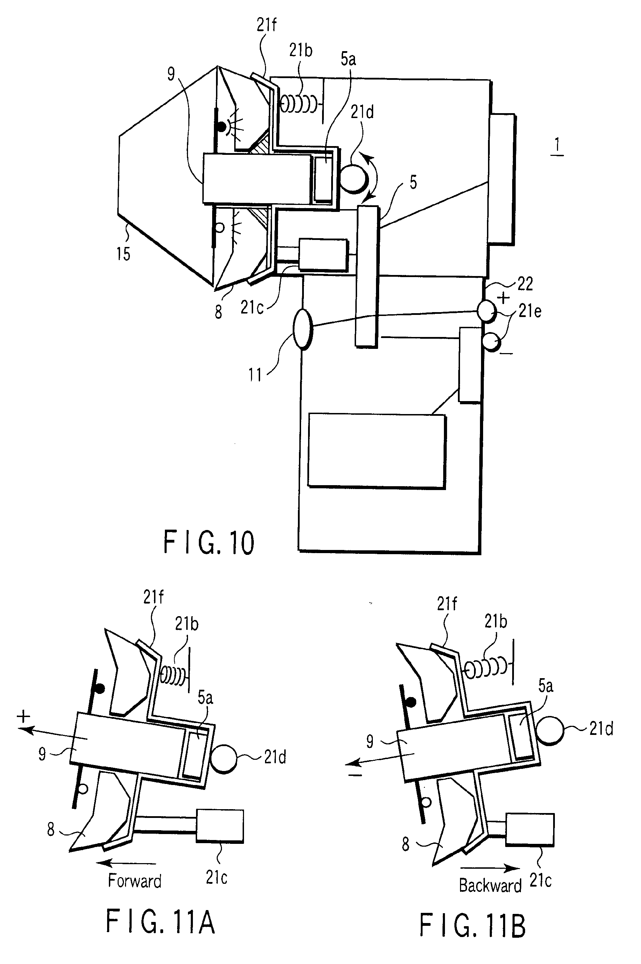

FIG. 10 shows the configuration of a camera unit 1 according to a fourth embodiment of the present invention. As shown in FIG. 10, a lighting optical unit 8, a photographing optics 9 and a CCD 5a required for observation are connected as one body on a holder plate 21f having a rotation adjusting means, so that they are placed opposite to a patient by a rotation adjusting motor 21c.

The lighting optical unit 8, photographing optics 9 and CCD 5a are connected on the holder plate 21f.
The holder plate 21f is fixed to a housing 22 rotatably about a rotation axis 21d. An adjustment spring 21b is connected to one end of the holder plate 21f. The other end of the adjustment spring 21b is connected to the housing 22. On the opposite side of the adjustment spring 21b viewed from the rotation axis 21d, the rotation adjusting motor 21c is fixed onto the housing 22. The rotation adjusting motor 21c is electrically connected to the electric board 5, so that the rotation adjusting motor 21c can be rotated forward and backward by a forward/backward rotation switch 21e.

To turn the holder plate 21f in the "+" direction, the observer presses the "+" position of the rotation switch 21e. A predetermined signal is sent from the rotation switch 21e to the rotation adjusting motor 21c, and the axis of the motor 21c is delivered. As a result, the holder plate 21f is pressed and turned in the "+" direction about the rotation axis 21d. The lighting optical unit 8, photographing optics 9 and CCD 5a are also turned together (FIG. 11A). The camera unit 1 is then placed opposite to the teeth of the patient.

FIG. 11B is a view showing that the observer presses one position of the rotation switch 21e to turn the holder plate 21f in one direction.

### (Embodiment 5)

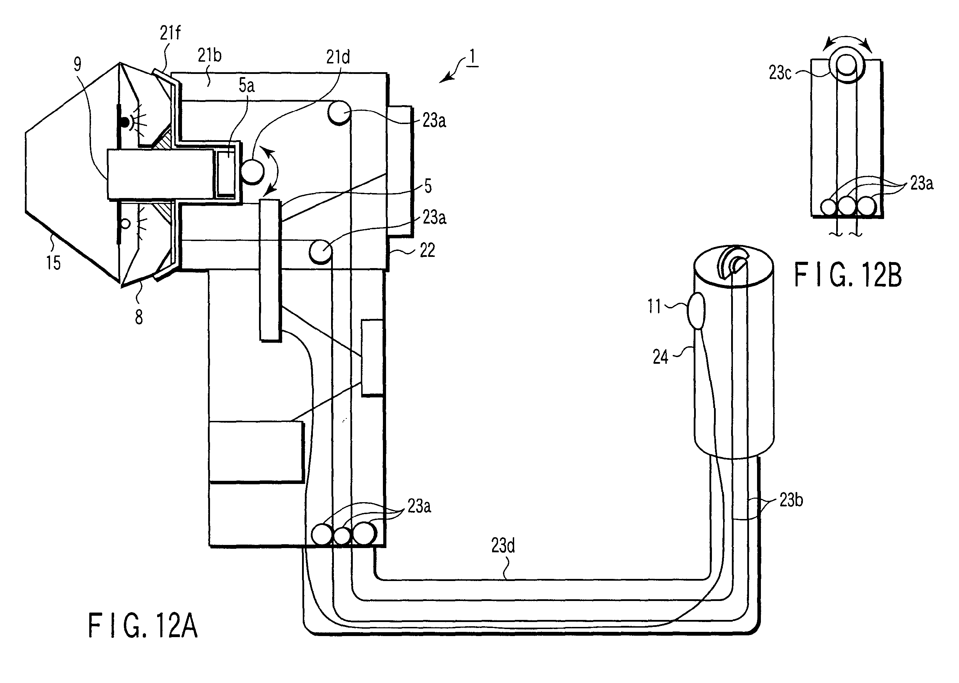

FIGS. 12A and 12B show the configuration of a camera unit 1 according to a fifth embodiment of the present invention. As shown in FIG. 12A, a lighting optical unit 8, a photographing optics 9 and a CCD 5a required for observation are connected as one body on a holder plate 21f having a rotation adjusting means, so that they are placed opposite to a patient by an external stick 24 provided outside of the camera unit 1.

The lighting optical unit 8, photographing optics 9 and CCD 5a are connected on the holder plate 21f.
The holder plate 21f is fixed to a housing 22 rotatably about a rotation axis 21d. One end of a wire 23d is connected one end of the holder plate 21f. The other end of the wire 23b is connected to the opposite side of the holder plate 21f.

The wire 23b is arranged by using pulleys 23a not to interrupt the parts of the camera unit 1. The wire 23b extending from the camera unit 1 can move freely in a wire cover 23d. The wire cover 23d is connected to an external stick 24. The wire 23b is arranged by using pulleys 23a not to be interrupted by the parts even in the external stick 24, and held by a jog 23c (FIG. 12B). In this embodiment, the shutter 11 is provided not in the camera unit 1 but in the external stick 24, thereby preventing a blur caused by moving the camera unit.

In the above configuration, the wire 23b is moved by turning the jog 23c forward and backward. This movement pulls one of the holders of the holder plate 21f. As a result, the holder plate 21f is turned about the rotation axis 21d, and the observer can place the lighting optical unit 8, photographing optics 9 and CCD 5a opposite to the patient.

In this embodiment, the external stick 24 is provided outside of the camera unit 1, and the shutter 11 is provided in the external stick 24. Therefore, the observer can hold the camera unit 1 by one hand and operate the external stick 24 by the other hand. The observer can place the unit opposite to the patient and operate the shutter, thereby improving the operability. To improve the operability furthermore, the rotation switch 21e for driving the rotation adjusting motor 21c may be provided in the external stick 24, as explained in the second embodiment.

### (Remarks)

According to the embodiments described hereinbefore, the invention having the following configurations can be extracted.

1. A mouthpiece used for a mouth photographing apparatus for photographing an oral cavity of a patient by shading external light, comprising:
a fitting portion which is used for fitting a shading means of the mouth photographing apparatus when taking a picture; and
a biting portion which positions the mouthpiece close to the teeth of a patient by being bitten by the patient with the teeth of upper and lower jaws.

### (Effect)

According to the mouthpiece described in the configuration 1, the mouthpiece is held by the biting portion in the oral cavity, the mouth photographing apparatus is applied to the mouthpiece fit to a tooth to be photographed, and light with a specific wavelength is applied to take a picture. The oral cavity of a patient can be exactly observed. The photographing apparatus is not directly touched to the gums of the patient, and the patient's load can be decreased.

2. The mouthpiece according to configuration 1, wherein the fitting portion has the characteristic to transmit substantially no light.

### (Effect)

The mouthpiece described in the configuration 2 does not transmit external light, and an unnecessary stray light is not applied to the teeth of a patient.

3. The mouthpiece according to configuration 1, further comprising a shading cover which adjoins to the fitting portion and presses the lips of a patient, and serves as a guide when the shading means of the mouth photographing apparatus is applied to the fitting portion, wherein the shading cover has the characteristic to transmit substantially no external light.

### (Effect)

The mouthpiece described in the configuration 3 fit with a shading cover of the mouth photographing apparatus does not transmit external light, and can have the function as a shading cover.

4. The mouthpiece according to configuration 1, wherein the biting portion is provided in two pieces on the left and right sides to be bitten by the upper/lower and left/right back teeth of a patient, and the circumference from the left to right biting portions is approximately 50 - 90 mm.

### (Effect)

The mouthpiece described in the configuration 4 permits biting with the teeth 4 - 6 ergonomically suitable for biting by setting the circumference from the left to right biting teeth to 50 - 90 mm. This permits to build up a system with a good holding stability.

5. The mouthpiece according to configuration 1, wherein the fitting portion is made of styrene group elastomer resin with the rubber hardness of approximately 20 - 70.

### (Effect)

The mouthpiece described in the configuration 5 can have stability and fitting easiness by being made of styrene group elastomer resin with the rubber hardness of 20 - 70.

6. The mouthpiece according to configuration 3, wherein the shading cover is made of styrene group elastomer resin with the rubber hardness of approximately 10 - 40.

### (Effect)

The mouthpiece described in the configuration 6 can have durability and fitting easiness by being made of styrene group elastomer resin with the rubber hardness of 10 - 40.

7. The mouthpiece according to configuration 2, wherein the color of at least the fitting portion is black.

### (Effect)

The mouthpiece described in the configuration 7 prevents the causes of error in measurement, such as, reflection of illumination light during photographing and a stray light reaching the CCD through the photographing optics, by being made of black material.

8. A mouth photographing apparatus for photographing the oral cavity of a patient by using the mouthpiece according to any one of configurations 1 - 7, comprising:
a lighting means to light the teeth of a patient;
a photographing means to photograph the teeth of a patient lit by the lighting means; and
a shading means to shade the photographing means from external light;
wherein the lighting means is positioned at a location capable of lighting the teeth of a patient substantially evenly by fitting the shading means to the fitting portion of the mouthpiece; and
the photographing means is positioned at a location capable of photographing the teeth of a patient in the state substantially focused.

### (Effect)

The mouth photographing apparatus described in the configuration 8 permits exact observation of the oral cavity of a patient, by applying a shading means to the fitting portion of the mouthpiece.

9. The mouth photographing apparatus according to configuration 8, wherein the lighting means and photographing means are configured as one body and rotatably about a predetermined rotation axis.

### (Effect)

The mouth photographing apparatus described in the configuration 9 places the lighting optical unit, photographing optics and CCD required for observation of the inside of the photographing apparatus opposite to the teeth of a patient by the rotation adjusting means, and can provide an exact reflected light from the teeth.

10. The mouth photographing apparatus according to configuration 9, further comprising:
a turning mechanism means to turn the lighting means and photographing means configured as one body; and
an operation means to adjust the turning amount of the turning mechanism means.

### (Effect)

The photographing apparatus described in the configuration 10 can be adjusted to a desired inclination by inclining the lighting means and photographing means by a turning mechanism.

11. The mouth photographing apparatus according to configuration 10, wherein the operation means is composed of a jog wheel and a wire moved by the rotation of the jog wheel, and the turning mechanism means turns the lighting means and photographing means configured as one body according to the movement of the wire.

### (Effect)

The photographing apparatus described in the configuration 11 can have an operation mechanism with a simple configuration, by being composed of a jog wheel and a wire.

### Industrial Applicability

A mouthpiece and a photographing apparatus, which can exactly observe the oral cavity of a patient and reduce the load of a patient, can be provided. A mouthpiece and a photographing apparatus with the improved operability can be provided.

## Claims

1. A mouthpiece used for a mouth photographing apparatus for photographing an oral cavity of a patient by shading external light, comprising:
a fitting portion which is used for fitting a shading means of the mouth photographing apparatus when taking a picture; and
a biting portion which positions the mouthpiece close to the teeth of a patient by being bitten by the patient with the teeth of upper and lower jaws.

2. The mouthpiece according to claim 1, wherein the fitting portion has the characteristic to transmit substantially no light.

3. The mouthpiece according to claim 1, further comprising a shading cover which adjoins to the fitting portion and presses the lips of a patient, and serves as a guide when the shading means of the mouth photographing apparatus is applied to the fitting portion, wherein the shading cover has the characteristic to transmit substantially no external light.

4. The mouthpiece according to claim 1, wherein the biting portion is provided in two pieces on the left and right sides to be bitten by the upper/lower and left/right back teeth of a patient, and the circumference from the left to right biting portions is approximately 50 - 90 mm.

5. The mouthpiece according to claim 1, wherein the fitting portion is made of styrene group elastomer resin with the rubber hardness of approximately 20 - 70.

6. The mouthpiece according to claim 3, wherein the shading cover is made of styrene group elastomer resin with the rubber hardness of approximately 10 - 40.

7. The mouthpiece according to claim 2, wherein the color of at least the fitting portion is black.

8. A mouth photographing apparatus for photographing the oral cavity of a patient by using the mouthpiece according to any one of claims 1 - 7, comprising:
a lighting means to light the teeth of a patient;
a photographing means to photograph the teeth of a patient lit by the lighting means; and
a shading means to shade the photographing means from external light;
wherein the lighting means is positioned at a location capable of lighting the teeth of a patient substantially evenly by fitting the shading means to the fitting portion of the mouthpiece; and
the photographing means is positioned at a location capable of photographing the teeth of a patient in the state substantially focused.

9. The mouth photographing apparatus according to claim 8, wherein the lighting means and photographing means are configured as one body and rotatably about a predetermined rotation axis.

10. The mouth photographing apparatus according to claim 9, further comprising:
a turning mechanism means to turn the lighting means and photographing means configured as one body; and
an operation means to adjust the turning amount of the turning mechanism means.

11. The mouth photographing apparatus according to claim 10, wherein the operation means is composed of a jog wheel and a wire moved by the rotation of the jog wheel, and the turning mechanism means turns the lighting means and photographing means configured as one body according to the movement of the wire.
